# EUROPEAN PATENT APPLICATION

(11) **EP 4 349 804 A1**
(43) Date of publication of application: **10.04.2024**
(21) Application number: 22810710.8
(22) Date of filing: 27.05.2022
(51) Int. Cl.: C07C 5/25, C07C 5/23, C07C 11/02, B01J 23/46

(54) **METHOD FOR OBTAINING LONG-CHAIN LINEAR ALKENES**

(30) Priority: 28.05.2021 ES 202130482
(71) Applicant: Consejo Superior De Investigaciones Científicas (CSIC), 28006 Madrid (ES); Universitat Politècnica de València, 46022 Valencia (ES)
(72) Inventor: MON CONEJERO, Marta, 46022 Valencia (ES); LEYVA PÉREZ, Antonio, 46022 Valencia (ES); OLIVER MESEGUER, Judit, 46022 Valencia (ES)
(74) Representative: Pons
(86) International application number: PCT/ES2022/070326
(87) International publication number: WO 2022/248758

(57) **Abstract**

The invention relates to a method for obtaining a linear, internal C₁₀-C₁₆ alkene from an unsubstituted linear, terminal C₁₀-C₁₆ alkene in the presence of a metal precursor.

## Description

The present invention relates to a method for generating long-chain linear alkenes, characterized in that they are useful for application as lubricants and in the paper industry.

### BACKGROUND OF THE INVENTION

Unsubstituted long-chain internal alkenes are essential chemical compounds in the industrial manufacturing chain, however, their synthesis remains costly and limits subsequent transformations.

This is because the most direct and easiest way to synthesize an internal alkene, i.e., migration of double bond to the desired internal position from the inexpensive and widely available terminal alkene to obtain a clean internal alkene, is still unresolved both at the research level and at the industrial level. Thus, the technology for the migration of double bond in alkenes still cannot meet the needs for preparing simple internal alkenes.

Currently, the isomerization of unsubstituted long-chain alkenes is performed in industry with basic solids, at temperatures between 200 and 450°C and in continuous flow, which generally produces a mixture of alkenes with some branched products after 80-90% conversion (Patent US 3723564, 1973; patent US 6281404 B1, 2001; Patent US 5801293 A1, 1998). In this mixture of different internal components, the expected migration positions (2, 3, 4, etc.) are obtained in addition to the corresponding cis and trans stereoisomers for each alkene (see comments below), providing a final mixture which may contain 2 (n - 1) isomers for an alkene with a given n alkyl chain. None of the earlier patents (see for example: US 5801293 A1) describes the final composition in detail. As an example, only the isomerization of short-chain gaseous alkenes such as 1-butene, described to date, allows a cis/trans ratio of 1: 2 in 2-butene after 80% conversion, which implies a poor yield of about 50% for pure trans-2-butene. This degree of conversion is not acceptable for many applications of the corresponding unsubstituted long-chain alkenes, such as lubrication or paper sizing. For example, long-chain lubricants require at least > 95% conversion (<5% starting terminal olefin), without any branching at the end product; otherwise, lubrication properties are lost and the desired end product becomes solid and does not remain liquid.

Current processes for the migration of double bond in petroleum-derived terminal alkenes utilize the production of flowing terminal alkenes, which is referred to as Normal Alpha Olefin (NAO) technology, to connect both processes. Examples of unsubstituted long-chain terminal alkenes, i.e., having more than eight carbons in order to be considered as linear long-chain alkenes, are not described in the state of the art (see, for example, US 8324423 B2).

Moreover, to date, terminal olefins from the NAO process require additional purification (by distillation) before entering the migration process, otherwise the catalyst is rapidly deactivated (US 3723564).

Furthermore, the migration of double bonds in unsubstituted long-chain terminal alkenes obtained from petrochemicals is not an optimized process, requires expensive catalysts, energy intensive processes, and results in incomplete conversions and product mixtures that include significant amounts of branched and saturated alkenes that are detrimental to the final mixture.

The isomerization of terminal olefins to internal olefins with flowing solid catalysts, achieving conversions of about 98%, using silica-alumina as a catalyst at 75-150°C (US 2005/0070747 A1), has also been described. As a result, the isomer distribution is rather wide, and the process requires recirculation of the product mixture for typically 4 hours in order to achieve a process yield of 20 kilograms of product per kilogram of catalyst, which is insufficient for a flexible, efficient, and inexpensive industrial production.

There are also studies on the use of metals supported on solids as catalysts for the migration of double bonds, including long-chain linear terminal alkenes (see US 3352939; and US 3409702). However, these solid catalysts require the use of mixtures of metals or aldehydes as additives, and in amounts of thousands of parts per million, with partial conversions (about 68%), which makes them not viable for industrial purposes.

Migration of double bonds in terminal alkenes has also been studied with homogeneous catalysts in solution. These catalysts can be basic or acidic.

KOH and NaOH in combination with iron (ChemSusChem 2012, 5, 734-739) or alumina (Eur. Pat. Appl. (1988), EP 279397 A1 19880824; Ger. Offen. (1972), DE 2137024 A 19720127) have been used as homogeneous catalysts in basic solution for the isomerization of simple alkenes. However, these bases must typically be used in very large (often stoichiometric) amounts, exhibit long reaction times, generate caustic residues, and give incomplete conversion in many cases. Furthermore, these strong bases are particularly unsuitable in the subsequent application of petroleum-derived long-chain linear internal alkenes, since any traces of base remaining in the final mixture will make the alkene unsuitable for subsequent acid-catalyzed reactions, such as, for example: hydro addition reactions, Friedel-Crafts reactions, or epoxidation reactions.

Soluble acidic catalysts based on metal complexes have been studied more than basic catalysts, and although these systems exhibit higher conversion and selectivity (up to 99% in both cases) than solid catalysts, and have opened new pathways for the synthesis of internal alkenes in laboratories, they introduce significant deterrents for industrial implementation, such as stoichiometric additives, large amount of solvent, and unacceptable metal catalyst load, typically 0.5-10 mol%, leading to unacceptable economics for a commercial industrial process (Science 363, 391-396, 2019; ChemCatChem 9, 3849-3859, 2017; Chem. Rev. 103, 27-51, 2003; J. Organomet. Chem. 86, C17, 1975; J. Mol. Cat. 11, 293-300, 1981). There are examples of Ru catalysts soluble in very low amounts for certain long-chain alkenes, however, they require the use of very expensive and toxic carbon monoxide complexes, in addition to the need for a carbon monoxide atmosphere in some cases, making these catalytic systems not only economically less attractive but also hazardous and undesirable when considering sustainability and safety aspects (WO2014/1508211 ; US 9708236 B2). Thus, it would be desirable to have a method that significantly improves the yield and selectivity of the migration of terminal double bonds to long-chain internal bonds in order to achieve their production on an industrial scale.

### DESCRIPTION OF THE INVENTION

In a first aspect, the invention relates to a method for obtaining a linear and internal C₁₀-C₁₆ alkene which comprises the following steps:
i) mixing a non-substituted linear terminal C₁₀-C₁₆ alkene with a supported or non-supported metal precursor; and
ii) heating the mixture obtained in step (i) at a temperature of between 150°C and 300°C, wherein the metal precursor gives rise to the *in situ* formation of isolated metal atoms which act as catalysts,
characterized in that:
- the metal precursor is used in an amount of less than 100 ppm by weight with respect to the unsubstituted linear terminal C₁₀-C₁₆ alkene.

Thus, the method of the present invention enables the generation of long-chain linear internal alkenes of between 10 and 16 unsubstituted and unbranched carbon atoms, preferably between 12 and 14 unsubstituted and unbranched carbon atoms, by means of the migration of the double bond in the corresponding terminal alkenes, in the presence of a supported or non-supported metal catalyst from groups VIII and IX in homeopathic amounts (less than 100 parts per million), in the absence of external reducing agents, such as H₂ or aldehydes, and in the absence of previously reduced soluble catalysts, such as hydrides or carbon monoxide. Moreover, the method is preferably carried out without solvent. C₁₀-C₁₆ alkenes are preferred for isomerization due to their unique hydrophobicity and density properties. Furthermore, the reaction takes place more efficiently compared to alkanes with a smaller or larger number of carbons because they cause reduced catalyst poisoning in both cases.

In another embodiment, the invention relates to the method described above, wherein steps (i) and (ii) are carried out in the absence of a solvent.

In another embodiment, the invention relates to the method described above, which further comprises an isolation step (iii) for isolating the product obtained in step (ii).

The isolation step (iii) referred to in the present invention consists of recovering the end product, since the elimination of an additive or by-product is not required.

In another embodiment, the invention relates to the method described above, wherein the unsubstituted linear terminal C₁₀-C₁₆ alkene of step (i) is an unsubstituted linear terminal C₁₂-C₁₄ alkene, and more preferably wherein the unsubstituted linear terminal C₁₀-C₁₆ alkene of step (i) is selected from 1-dodecene and 1-tetradecene.

In another embodiment, the invention relates to the method described above, wherein the metal precursor of step (i) is selected from salts, complexes, nanoparticles, and macroscopic forms of metals from groups VIII and IX of the periodic table or a mixture thereof.

In another embodiment, the invention relates to the method described above, wherein the metal of the metal precursor of step (i) is from group VIII of the periodic table, and preferably wherein the metal of the metal precursor of step (i) is from group VIII of the periodic table and is selected from Fe, Ru, Os, and a mixture thereof.

In another embodiment, the invention relates to the method described above, wherein the metal of the metal precursor of step (i) is from group IX of the periodic table, and preferably wherein the metal of the metal precursor of step (i) is from group IX of the periodic table and is selected from Co, Rh, Ir, and a mixture thereof.

In another embodiment, the invention relates to the method described above, wherein the metal of the metal precursor of step (i) is selected from Fe, Ru, Os, Co, Rh, Ir, and combinations thereof, preferably selected from Fe, Ru, and a mixture thereof, and more preferably wherein the metal precursor of step (i) is Ru.

In another embodiment, the invention relates to the method described above, wherein the metal of the metal precursor of step (i) is Ru(III), Ru(II), or Ru(0).

In another embodiment, the invention relates to the method defined above, wherein the metal precursor is selected from Ru₃(CO)₁₂, RuCl₃, Ru(C₄H₈)₂COD, Ru(PPh)₃Cl₂, Ru nanoparticles in colloidal form, and Ru nanoparticles as a pure metal.

Throughout the invention, nanoparticles (NPs) in colloidal form refer to surfactant-stabilized nanoparticles. Examples include, among others, carboxylic acids, carbenes, thiols, and ammonium salts.

Nanoparticles (NPs) as a pure metal refer to nanoparticles that must be supported.

In another embodiment, the invention relates to the method defined above, wherein the metal precursor is supported on inorganic oxides, and preferably wherein the inorganic oxides are selected from alumina, titania, silica, zinc oxide, zirconium oxide, nanoceria; activated carbon, and/or combinations thereof.

In another embodiment, the invention relates to the method defined above, wherein the metal precursor is a Ru salt which is optionally reduced.

In another embodiment, the invention relates to the method defined above, wherein the metal precursor is supported on activated carbon.

In another embodiment, the invention relates to the method described above, wherein the unsubstituted linear terminal C₁₀-C₁₆ alkene of step (i) is present in an amount of between 10,000 equivalents and 100,000,000 equivalents with respect to the metal precursor, preferably between 1,000,000 equivalents and 10,000,000 equivalents with respect to the metal precursor, and more preferably between 2,000,000 equivalents and 5,000,000 equivalents with respect to the metal precursor.

In another embodiment, the invention relates to the method defined above, wherein the metal precursor is used in an amount of between 1 ppm and 100 ppm by weight with respect to the unsubstituted linear terminal C₁₀-C₁₆ alkene, preferably between 1 ppm and 50 ppm by weight with respect to the unsubstituted linear terminal C₁₀-C₁₆ alkene, and more preferably between 1 and 10 ppm by weight with respect to the unsubstituted linear terminal C₁₀-C₁₆ alkene.

In another embodiment, the invention relates to the method defined above, wherein step (ii) of the method is carried out in a batch-type reactor with simple stirring or in a tank-type continuous reactor stirred with a continuous flow or fixed bed, and preferably in a batch-type reactor with simple stirring.

Throughout the invention, the term "batch" refers to a stationary batch reactor.

In another embodiment, the invention relates to the method defined above, wherein the temperature of step (ii) is between 200°C and 250°C, and more preferably at a temperature of 200°C.

In another embodiment, the invention relates to the method defined above, wherein step (ii) is carried out at a pressure of between 1 bar and 20 bar, and preferably 1 bar.

In another embodiment, the invention relates to the method defined above, wherein step (ii) is carried out under inert or ambient atmosphere, preferably under inert nitrogen, helium, or argon atmosphere, more preferably under nitrogen atmosphere.

Throughout the invention, the term "ambient atmosphere" refers to carrying out same in an open air environment, not under inert atmosphere.

In another embodiment, the invention relates to the method defined above, wherein step (ii) is carried out in the presence of organic solvents, and preferably in the presence of aromatic solvents.

In another embodiment, the invention relates to the method defined above, wherein the reaction time of step (ii) is between 0.5 h and 72 h, and preferably between 1 h and 24 h.

In another embodiment, the invention relates to the method defined above, wherein:
the unsubstituted linear terminal C₁₀-C₁₆ alkene of step (i) is 1-dodecene;
the metal precursor of step (i) is Ru; and
the temperature of step (ii) is 200°C.

In another embodiment, the invention relates to the method defined above, wherein:
the unsubstituted linear terminal C₁₀-C₁₆ alkene of step (i) is 1-dodecene;
the metal precursor of step (i) is Ru;
the temperature of step (ii) is 200°C; and
step (ii) is carried out under inert nitrogen atmosphere.

In another embodiment, the invention relates to the method defined above, wherein:
the unsubstituted linear terminal C₁₀-C₁₆ alkene of step (i) is 1-dodecene;
the metal precursor of step (i) is Ru;
the temperature of step (ii) is 200°C;
step (ii) is carried out under inert nitrogen atmosphere;
reaction time is 4.5 h; and
the reaction pressure is 1 bar.

In another embodiment, the invention relates to the method defined above, wherein:
the unsubstituted linear terminal C₁₀-C₁₆ alkene of step (i) is 1-tetradecene;
the metal precursor of step (i) is Ru.

In another embodiment, the invention relates to the method defined above, wherein:
the unsubstituted linear terminal C₁₀-C₁₆ alkene of step (i) is 1-tetradecene;
the metal precursor of step (i) is Ru; and
the temperature of step (ii) is 200°C.

In another embodiment, the invention relates to the method defined above, wherein:
the unsubstituted linear terminal C₁₀-C₁₆ alkene of step (i) is 1-tetradecene;
the metal precursor of step (i) is Ru;
the temperature of step (ii) is 200°C;
step (ii) is carried out under inert nitrogen atmosphere;
reaction time is 5 h; and
the reaction pressure is 1 bar.

Throughout the present invention, the term "unsubstituted linear terminal C₁₀-C₁₆ alkene" refers to an alkene having 10 to 16 carbon atoms which does not have branching or substitution and having a terminal double bond. Examples include 1-decene, 1-undecene, 1-dodecene, 1-tridecene, 1-tetradecene, 1-pentadecene, and 1-hexadecene.

The term "metal precursor" refers to salts, complexes, nanoparticles, and macroscopic forms of metals which may be supported on a solid or may not be supported on a solid, which may give rise to the *in situ* formation of extremely active isolated metal atoms that are useful as catalysts for carrying out the migration of the double bond of the unsubstituted linear terminal C₁₀-C₁₆ alkene without having to use solvents. The solid support may be an inorganic oxide such as alumina, zeolites, or silica. Examples include, among others, metals from groups VIII and IX of the periodic table, such as, Fe, Ru, Os, Co, Rh, and Ir.

The catalyst formed in situ which is used in the method of the invention is based on the use of extremely small amounts, typically parts per million (ppm).

The structural characteristics of unsubstituted linear internal C₁₀-C₁₆ alkenes which are obtained by the method of the invention make them useful in the industry of lubricants and paper processing.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

**Figure 1** shows the 1H-NMR spectrum of the starting 1-dodecene (A) and the reaction product (B) after 4.5 h of reaction with 0.0005 mol% of Ru(C₄H₈)₂(COD).
**Figure 2** shows the 1H-NMR spectrum of the starting 1-tetradecene (A) and the reaction product (B) after 4.5 h of reaction with 0.0005 mol% of Ru(C₄H₈)₂(COD).

### EXAMPLES

Next, the invention will be illustrated by means of assays carried out by the inventors that demonstrate the effectiveness of the product of the invention.

Table 1 shows the results with 1-dodecene for a Ru-type catalyst. Migration of the double bond occurs with only 5 ppm by weight of Ru catalyst (entry 6) at >95% conversion, although with a long reaction time (20 h). This linear terminal olefin with alkyl chain has a boiling point above 200°C and a good thermal stability, which allows the possible conversion thereof to internal alkenes at temperatures above 150°C and with improved conversions in less time. Table 1 also shows that, at 200°C, isomerization continues at >97% conversions after 4.5 h. In this case, it should be mentioned that, depending on the amount of catalyst, the internal alkene mixture is different. For example, there are 72% of 2-dodecene and 18% of other more internal alkenes by using 1 ppm by weight of catalyst, and 26% of 2-dodecene with 5 ppm by weight. The results are consistent for starting materials (1-dodecene) of several commercial companies. In any case, the final linear internal alkene mixture is consistent for a given amount of catalyst.

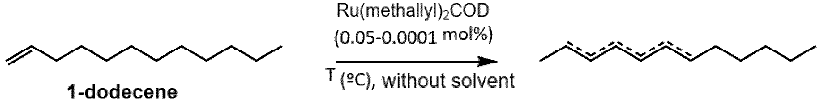

**Table 1**

| **T = 150°C** | | | | | | **T = 200°C** | | | | |
|---|---|---|---|---|---|---|---|---|---|---|
| **Exp.** | **Conditions** | | **Conv.** | **Yield (%)** | | **Conditions** | | **Conv** | **Yield (%)** | |
| | **(mol%)** | **t(h)** | **(%)** | **2-alkene** | **Others** | **(mol%)** | **t (h)** | **.(%)** | **2-alkene** | **others** |
| **1** | 0.05 | 2 | 100.0 | 20.2 | 79.8 | **0.0005^{a}** | **4.5** | **97.3** | **26.0** | **71.3** |
| **2** | 0.01 | 2 | 100.0 | 51.2 | 48.8 | 0.00025 | 10 | 95.6 | 48.7 | 46.9 |
| **3** | 0.001 | 2 | 91.8 | 79.0 | 12.8 | 0.0001 | 17 | 90.5 | 72 | 18.5 |
| **4** | | 20 | 100.0 | 29.4 | 70.6 | | | | | |
| **5** | 0.0005 | 2 | 88.3 | 78.9 | 9.4 | | | | | |
| **6** | | 20 | 95.6 | 49.4 | 46.2 | | | | | |
| **7** | 0.0001 | 21 | 83.5 | 76.6 | 6.9 | | | | | |

Table 2 shows the results for 1-tetradecene at reaction temperatures of 150 and 200°C. As evaluated above for 1-dodecene, higher conversions are obtained in less time at 200°C, with >97% conversion with 5 ppm by weight of Ru after 5 h. There is a need to take into account that a >95% conversion is difficult to achieve with any industrial method and is highly beneficial for the final application of the product.

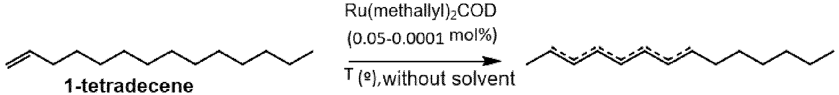

**Table 2**

| **T = 150°C** | | | | | | **T = 200°C** | | | | |
|---|---|---|---|---|---|---|---|---|---|---|
| **Exp .** | **Conditions** | | **Conv .(%)** | **Yield (%)** | | **Conditions** | | **Conv . (%)** | **Yield (%)** | |
| | **(mol%)** | **t (h)** | | **2-alkene** | **others** | **(mol%)** | **t(h)** | | **2-alkene** | **others** |
| **1** | 0.05 | 2 | 100.0 | 33.5 | 66.5 | **0.0005** | 5 | **97.7** | **33.5** | **64.2** |
| **2** | 0.01 | 2 | 100.0 | 77.9 | 22.1 | 0.00025 | 22 | 86.7 | 65.2 | 21.5 |
| **3** | 0.001 | 5 | 90.5 | 75.5 | 15.0 | 0.0001 | 22 | 66.7 | 59.6 | 7.1 |
| **4** | | 20 | 100.0 | 3.3 | 60.7 | | | | | |
| **5** | 0.0005 | 5 | 89.6 | 78.8 | 10.8 | | | | | |
| **6** | | 21 | 95.6 | 44.5 | 51.1 | | | | | |
| **7** | 0.0001 | 21 | 54.5 | 52.0 | 2.5 | | | | | |

### Example 1: Method for the migration of a double bond in 1-dodecene with 5 ppm by weight of the catalyst Ru(C₄H₈)₂COD

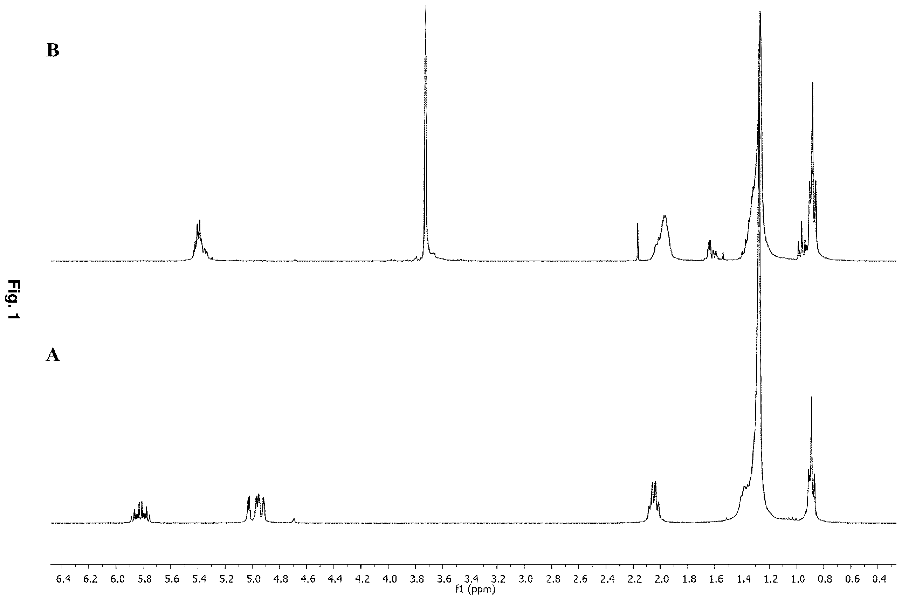

1-Dodecene (27 g) was charged into a 50 ml flask equipped with a magnetic stirrer and the Ru(methallyl)₂COD catalyst (0.0005 mol%) was added. The flask was closed with a septum, nitrogen atmosphere was created, and the flask was placed in a preheated oil bath at 200°C under magnetic stirring for a given reaction time. Aliquots were taken from the reaction mixture to monitor the reaction over time by means of GC and NMR. It should be noted that stock solutions of the Ru catalyst in dichloromethane (which evaporates during reaction) were prepared, since the amounts used are too small to be weighed. To prepare these solutions, volumetric flasks were used with dichloromethane as solvent. For GC analysis, 5.6 µl of the reaction mixture were diluted with 1 ml of ethyl acetate. For NMR analysis, 20 mg of the reaction mixture were dissolved in deuterated chloroform (CDCl₃ signal: singlet, 7.26 ppm), 15 mg of 1,2-dichloroethane were also added (signal: singlet, 3.73 ppm) as an internal standard for calculating the conversion of the reaction. Figure 1 shows how the starting terminal alkene disappears and the desired products appear.

### Example 2: Method for the migration of double bond in 1-tetradecene with 5 ppm by weight of the catalyst Ru(C₄H₈)₂COD

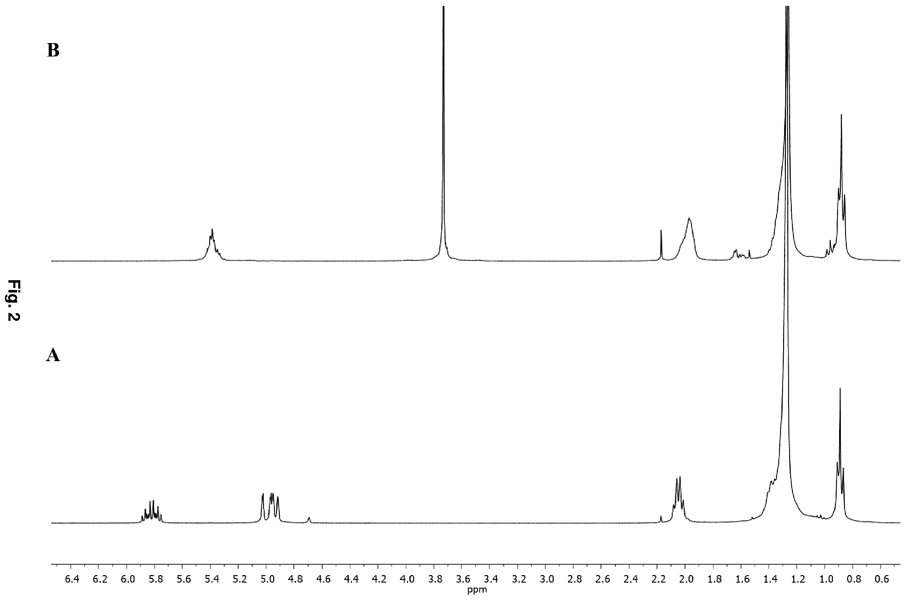

1-Tetradecene (32 g) was charged into a 50 ml flask equipped with a magnetic stirrer and the Ru(methallyl)₂COD catalyst (0.0005 mol%) was added. The flask was closed with a septum, nitrogen atmosphere was created, and the flask was placed in a preheated oil bath at 200°C under magnetic stirring for a given reaction time. Aliquots were taken from the reaction mixture to monitor the reaction over time by means of GC and NMR. It should be noted that stock solutions of the Ru catalyst in dichloromethane (which evaporates during reaction) were prepared, since the amounts used are too small to be weighed. To prepare these solutions, volumetric flasks were used with dichloromethane as solvent. For CG analysis, 5.6 µl of the reaction mixture were diluted with 1 ml of ethyl acetate. For NMR analysis, 20 mg of the reaction mixture were dissolved in deuterated chloroform (CDCl₃ signal: singlet, 7.26 ppm), 15 mg of 1,2-dichloroethane were also added (signal: singlet, 3.73 ppm) as an internal standard for calculating the conversion of the reaction. Figure 2 shows how the starting terminal olefin disappears and the desired products appear.

### Example 3: Method for the migration of a double bond in 1-dodecene in a stirred reactor with a solid Ru catalyst on silica

A solid Ru catalyst on silica, prepared by the impregnation of RuCl₃ on silica with a large surface area for a final load of 1% by weight of Ru, was added into a 50 ml flask equipped with a magnetic stirrer so as to achieve 0.001 mol% with respect to 1-dodecene (27 g), which was added later. The flask was closed with a septum, nitrogen atmosphere was created, and the flask was placed in a preheated oil bath at 200°C under magnetic stirring for a given reaction time. Supernatant samples were taken to monitor the reaction over time by means of GC and NMR. For NMR analysis, 20 mg of the supernatant were dissolved in deuterated chloroform (CDCl₃ signal: singlet, 7.26 ppm), 15 mg of 1,2-dichloroethane were also added (signal: singlet, 3.73 ppm) as an internal standard for calculating the conversion of the reaction.

## Claims

1. A method for generating an internal and linear C₁₀-C₁₆ alkene which comprises the following steps:
i) mixing an unsubstituted linear terminal C₁₀-C₁₆ alkene with a supported or non-supported metal precursor; and
ii) heating the mixture obtained in step (i) at a temperature of between 150°C and 300°C, wherein the metal precursor gives rise to the *in situ* formation of isolated metal atoms which act as catalysts,
**characterized in that**:
- the metal precursor is used in an amount of less than 100 ppm by weight with respect to the unsubstituted linear terminal C₁₀-C₁₆ alkene, wherein:
steps (i) and (ii) are carried out in the absence of a solvent, and
the metal of the metal precursor of step (i) is Ru.

2. The method according to claim 1, wherein the unsubstituted linear terminal C₁₀-C₁₆ alkene of step (i) is an unsubstituted linear terminal C₁₂-C₁₄ alkene.

3. The method according to any of claims 1 or 2, wherein the unsubstituted linear terminal C₁₂-C₁₄ alkene of step (i) is selected from 1-dodecene and 1-tetradecene.

4. The method according to any of claims 1 to 3, wherein the metal precursor is selected from Ru₃(CO)₁₂, RuCl₃, Ru(C₄H₈)₂COD, Ru(PPh)₃Cl₂, Ru nanoparticles in colloidal form, and Ru nanoparticles as a pure metal.

5. The method according to any of claims 1 to 4, wherein the metal precursor is supported on inorganic oxides.

6. The method according to any of claims 1 to 5, wherein the unsubstituted linear terminal C₁₀-C₁₆ alkene of step (i) is present in an amount of between 10,000 equivalents and 100,000,000 equivalents with respect to the metal precursor.

7. The method according to any of claims 1 to 6, wherein the metal precursor is used in an amount of between 1 ppm and 100 ppm by weight with respect to the unsubstituted linear terminal C₁₀-C₁₆ alkene.

8. The method according to any of claims 1 to 7, wherein step (ii) is carried out in a batch-type reactor with simple stirring or in a tank-type continuous reactor stirred with a continuous flow or fixed bed.

9. The method according to any of claims 1 to 8, wherein the temperature of step (i) is between 200°C and 250°C.

10. The method according to any of claims 1 to 9, wherein step (ii) is carried out at a pressure of between 1 bar and 20 bar.

11. The method according to any of claims 1 to 10, wherein step (ii) is carried out under an inert atmosphere.

12. The method according to any of claims 1 to 11, wherein the reaction time of step (ii) is between 0.5 h and 72 h.
